# EUROPÄISCHE PATENTSCHRIFT

(11) **EP 1 002 122 B1**
(45) Veröffentlichungstag und Bekanntmachung des Hinweises auf die Patenterteilung: **19.03.2003**
(21) Anmeldenummer: 98941398.4
(22) Anmeldetag: 22.07.1998
(51) Int. Cl.: C12P 13/02, C12N 1/20, C12N 9/88

(54) **VERFAHREN ZUR HERSTELLUNG VON AMIDEN**
PROCESS FOR PREPARING AMIDES
PROCEDE DE FABRICATION D'AMIDES

(30) Priorität: 22.07.1997 CH 177697; 17.11.1997 CH 262997; 06.04.1998 CH 81598
(43) Veröffentlichungstag der Anmeldung: 24.05.2000
(73) Patentinhaber: LONZA A.G., CH-4002 Basel (CH)
(72) Erfinder: ROBINS, Karen, Tracey, South Guangzhou 510288 (CN); NAGASAWA, Toru, Gifu 501-11 (JP)
(74) Vertreter: Winter, Brandl, Fürniss, Hübner, Röss, Kaiser, Polte Partnerschaft
(86) Internationale Anmeldenummer: EP9804587
(87) Internationale Veröffentlichungsnummer: WO99005306

(56) Entgegenhaltungen:
- EP-A- 0 188 316
- EP-A- 0 307 926
- EP-A- 0 362 829
- GB-A- 2 290 295
- US-A- 5 200 331
- CHEMICAL ABSTRACTS, vol. 120, no. 13, 28. März 1994 Columbus, Ohio, US; abstract no. 161780, WAKAMOTO, AKIKO ET AL: "Manufacture of racemic 2-aminonitriles with bacteria" XP002085191 & JP 05 284982 A (NIKKO KYOSEKI KK, JAPAN)

## Beschreibung

Die Erfindung betrifft neue Mikroorganismen der Gattung Actinomadura, Amycolatopsis und Rhodococcus sowie ein neues Verfahren zur Herstellung von Amiden unter Verwendung dieser Mikroorganismen bzw. unter Verwendung von Enzymextrakten dieser Mikroorganismen.

Für die Herstellung von Amiden, wie beispielsweise Nikotinsäureamid, einem für Tier und Mensch essentiellen Vitamin des Vitamin-B-Komplexes, sind bereits mehrere biotechnologische Verfahren bekannt. Generell ist bekannt, dass Nitrilhydratase enthaltende Mikroorganismen Nitrile zu den entsprechenden Amiden umsetzen. So beschreibt die EP-A-0 188 316 ein Verfahren zur Herstellung von Nikotinsäureamid ausgehend von 3-Cyanpyridin unter Verwendung von Mikroorganismen der Gattungen Rhodococcus, Arthrobacter oder Mikrobacterium. Nachteilig bei diesem Verfahren ist, dass diese Mikroorganismen für die Umsetzung von 3-Cyanpyridin zum Nikotinsäureamid nur eine geringe Aktivität aufweisen.
Die EP-A-0 307 926 beschreibt die Umsetzung von 3-Cyanpyridin zum Nikotinsäureamid mittels Mikroorganismen der Spezies Rhodococcus rhodochrous J1. Damit diese Mikroorganismen die gewünschte Umsetzung katalysieren, müssen sie induziert werden. Ein weiterer Nachteil dieses Verfahrens ist, dass Rhodococcus rhodochrous J1 rot gefärbt ist und demzufolge eine Verfärbung des Produktes stattfindet. Desweiteren weist dieser Mikroorganismus eine geringe Hitzestabilität auf und wird z.B. durch das Substrat 3-Cyanpyridin inhibiert.
Ein weiteres Verfahren zur Herstellung von Nikotinsäureamid ausgehend von 3-Cyanpyridin mittels Mikroorganismen der Spezies Rhodococcus rhodochrous J1 ist in der EP-A-0 362 829 beschrieben. Um die spezifische Aktivität der Nitrilhydratase enthaltenden Mikroorganismen zu erhöhen, wurde zum Kultivierungsmedium Harnstoff bzw. ein Harnstoffderivat als Induktor zugegeben. Wie bei dem zuvor beschriebenen Verfahren findet auch bei diesem eine Verfärbung des Produktes statt.
Desweiteren beschreibt die WO 95/17 505 ein Verfahren zur Herstellung von aromatischen Amiden ausgehend von den entsprechenden Nitrilen mittels Mikroorganismen der Spezies Rhodococcus rhodochrous M33. Nachteilig bei diesem Verfahren ist die Rotfärbung von Rhodococcus rhodochrous M33 sowie der hohe K_{M}-Wert für das Substrat 3-Cyanpyridin. Die US-A-5 200 331 beschreibt die Umsetzung von Nitrilen zu Amiden mit verschiedenen Mikroorganismen der Gattung Rhodococcus.

Aufgabe der vorliegenden Erfindung war es, diese Nachteile zu beseitigen und ein Verfahren zur Herstellung von Amiden zur Verfügung zu stellen, bei dem die entsprechenden Amide in guter Ausbeute und Reinheit isoliert werden können.

Diese Aufgabe wird mit den neuen Mikroorganismen gemäss Anspruch 1 und 3 sowie mit dem Verfahren gemäss Anspruch 6 gelöst.

Erfindungsgemäss wird das Verfahren derart durchgeführt, daß die Umsetzung des Nitrils, als Substrat, zum entsprechenden Amid mit Mikroorganismen der Gattungen Actinomadura oder Amycolatopsis oder mit Mikroorganismen der Spezies Rhodococcus GF270 und GF376, mit einem Enzymextrakt von diesen Mikroorganismen oder mit gereinigter Nitrilhydratase aus Mikroorganismen der Gattungen Amycolatopsis oder Actinomadura erfolgt.

Die für die Biotransformation eingesetzten Nitrile, wie z. B. 3-Cyanpyridin, sind käufliche Verbindungen.

Die erfindungsgemäßen Mikroorganismen sind in der Lage, Nitrile als Substrate in die entsprechenden Amide zu überführen. Bevorzugt besitzen diese Mikroorganismen die Fähigkeit, auf Nitrilen oder Amiden als einziger C- und/oder N-Quelle zu wachsen.

Die erfindungsgemäßen Mikroorganismen sind durch geeignete Selektion beispielsweise aus Bodenproben, Schlamm oder Abwasser unter Zuhilfenahme üblicher mikrobiologischer Techniken erhältlich. Zweckmäßig werden die Mikroorganismen durch Anzucht mit Nitrilen oder Amiden als bevorzugt einziger C- und N-Quelle in Anwesenheit von Cobaltionen selektioniert. Zur Selektion geeignete Nitrile und Amide sind insbesondere die auch bei der späteren Biotransformation als Substrate eingesetzten Nitrile und die entsprechenden, daraus erhältlichen Amide. Geeignete Anzuchtmedien sind dem Fachmann ebenfalls bekannt; beispielsweise kann das in Tabelle 1 beschriebene Medium verwendet werden.

Üblicherweise werden die Mikroorganismen in gleicher Weise auch vor der eigentlichen Biotransformation kultiviert (angezogen), wobei die oben genannten Medien zur Anwendung kommen.

Wie fachmännisch bekannt, wird eine Ntrilhydratase nur gebildet, wenn das Anzuchtmedium Cobaltionen als Cofaktor enthält. Geeignete "Cobaltionen generierende Cobaltverbindungen" sind Co²⁺- oder Co³⁺-Salze . Beispiele für Co²⁺- und Co³⁺-Salze sind Cobaltchloride, Cobaltsulfate und Cobaltacetate.
Zweckmässig wird als Cobaltverbindung ein Co²⁺-Salz wie z. B. CoCl₂ eingesetzt. Die Anzucht kann jedoch auch in Gegenwart von Vitamin B 12 zusammen mit metallischem Cobalt oder anderen Cobaltverbindungen durchgeführt werden, die in situ ein Cobaltion erzeugen. Zweckmässig wird die Cobaltverbindung in einer Menge von 1 bis 10 mg/l vorzugsweise von 1 bis 3 mg/l eingesetzt.

Üblicherweise erfolgt die Anzucht bei einer Temperatur von 20 bis 50 °C und bei einem pH-Wert zwischen pH 5 und pH 8, vorzugsweise von 30 bis 45 °C und zwischen pH 5,5 und pH 7,5.

Die eigentliche Biotransformation kann mit Mikroorganismen der Gattung Actinomadura, Amycolatopsis, mit einem Enzymextrakt von diesen Mikroorganismen oder mittels gereinigter Nitrilhydratase aus diesen Mikroorganismen erfolgen. Zweckmässig erfolgt die Biotransformation mit Mikroorganismen der Spezies Actinomadura spadix, beispielsweise den Isolaten Actinomadura spadix E3733, Actinomadura spadix E3736, Actinomadura spadix 45A32, Actinomadura spadix 4501 oder Actinomadura spadix C15. Bevorzugt erfolgt die Biotransformation mit Mikroorganismen entsprechend den Spezies Amycolatopsis NE 31 und Amycolatopsis NA40 oder deren funktionell äquivalenten Varianten und Mutanten. Besonders bevorzugt werden Mikroorganismen entsprechend der Spezies Amycolatopsis NA40 eingesetzt. Mikroorganismen der genannten Spezien wurden am 03.06.1997 bei der Deutschen Sammlung von Mikroorganismen und Zellkulturen GmbH, Mascheroderweg 1b, D-38124 Braunschweig gemäss Budapester Vertrag unter den Bezeichnungen Amycolatopsis NE 31 und Amycolatopsis NA40 hinterlegt und haben die Hinterlegungsnummern DSMZ 11616 bzw. DSMZ 11617 erhalten. Diese beiden Mikroorganismen wurden genauer identifiziert und sind in der Literatur noch nicht beschriebenen Spezien der Gattung Amycolatopsis zuzuordnen.

Demzufolge betrifft die Erfindung auch Mikroorganismen der Gattung Amycolatopsis oder Actinomadura, die befähigt sind, ein Nitril in ein Amid umzusetzen, insbesondere Mikroorganismen mit der Bezeichnung Amycolatopsis NA40 (DSMZ 11617) und Amycolatopsis NE31 (DSMZ 11616).

Desweiteren wurde gefunden, dass spezielle Mikroorganismen der Gattung Rhodococcus bessere Eigenschaften für die Umsetzung von Nitrilen zu Amiden aufweisen als der in der EP-A-0 362 829 beschriebene Rhodococcus rhodochrous J1. Diese Mikroorganismen sind Rhodococcus GF270 (DSMZ 12211) und Rhodococcus GF376 (DSMZ 12175) oder deren funktionell äquivalente Varianten und Mutanten. Der Mikroorganismus DSMZ 12175 wurde am 15.5.1998 und der Mikroorganismus DSMZ 12211 am 8.6.1998 bei der Deutschen Sammlung von Mikroorganismen und Zellkulturen GmbH gemäss Budapester Vertrag hinterlegt.
Die Rhodococcus-Stämme GF270 und GF376 sind gemäss Identifikation in der Literatur noch nicht beschriebenen Spezien der Gattung Rhodococcus zugeordnet worden. Demzufolge betrifft die Erfindung auch die Mikroorganismen Rhodococcus GF270 und Rhodococcus GF376.
Im Gegensatz zu den Mikroorganismen der Gattung Actinomadura bzw. Amycolatopsis werden die Mikroorganismen der Gattung Rhodococcus zweckmässig vor der eigentlichen Umsetzung induziert. Als Induktoren sind die in der EP-A-0 307 926 beschriebenen Induktoren geeignet wie beispielsweise Acetamid, Buttersäureamid, Methacrylamid, Propionsäureamid, Crotonamid und Valeriansäureamid.

Unter "funktionell äquivalente Varianten und Mutanten" werden Mikroorganismen verstanden, die sich von den oben erwähnten Ursprungsorganismen ableiten und im wesentlichen dieselben Eigenschaften und Funktionen wie diese besitzen. Derartige Varianten und Mutanten können zufällig, z. B. durch UV-Bestrahlung oder durch mutagene Chemikalien gebildet werden.

| **Identifizierung von Amycolatopsis NA40** | |
|---|---|
| Farbe Luft-Myzel | weiss |
| Farbe Substrat-Myzel | orange |
| Farbe diffund. Pigment | - |

| Zuckerspektrum | |
|---|---|
| ARA | + |
| GAL | + |
| MAD | - |
| XYL | - |
| GLU | tr |
| RIB | + |
| Typ | A |
| DAP | DL |

| Menachinone (in %) | |
|---|---|
| 8/4 | - |
| 9/0 | (+) |
| 9/2 | + |
| 9/4 | +++ |
| 9/6 | - |
| 9/8 | - |
| 16S rDNA Homologie | 96,9 % |
| Phospholipide wie | nicht untersucht |
| PE, OH-PE, lyso PE, met PE, PC, NPG, PI, PIM, PG, DPG, GL | |

| Fettsäuren | |
|---|---|
| iso 16 | +++ |
| iso 15 | + |
| iso 17 | (+) |
| Anteiso 15 | (+) |
| Anteiso 17 | (+) |
| 10-Me 16 | - |
| 10-Me 17 | + |
| 2-OH 15 | + |
| 2-OH 16 | + |
| Typ | 3f |
| MS | - |

| **Identifizierung von Amycolatopsis NE31** | |
|---|---|
| Farbe Luft-Myzel | weiss |
| Farbe Substrat-Myzel | orange |
| Farbe diffund. Pigment | - |

| Zuckerspektrum | |
|---|---|
| ARA | + |
| GAL | + |
| MAD | - |
| XYL | - |
| GLU | tr |
| RIB | + |
| Typ | A |
| DAP | DL |

| Menachinone (in %) | |
|---|---|
| 8/4 | - |
| 9/0 | (+) |
| 9/2 | + |
| 9/4 | +++ |
| 9/6 | - |
| 9/8 | - |
| 16S rDNA Homologie | 96,1 % |
| Phospholipide wie | nicht untersucht |
| PE, OH-PE, lyso PE, met PE, PC, NPG, PI, PIM, PG, DPG, GL | |

| Fettsäuren | |
|---|---|
| iso 16 | +++ |
| iso 15 | + |
| iso 17 | (+) |
| Anteiso 15 | (+) |
| Anteiso 17 | (+) |
| 10-Me 16 | - |
| 10-Me 17 | + |
| 2-OH 15 | + |
| 2-OH 16 | + |
| Typ | 3f |
| MS | - |

| **Abkürzungen und Erläuterungen zur Identifizierung** | |
|---|---|
| (+) | 1 - 5 % |
| + | 5 - 15 % |
| ++ | 15 - 30 % |
| +++ | >30 % |
| DAP | Diaminopimelinsäure |
| ARA | Arabinose |
| GAL | Galaktose |
| MAD | Madurose |
| XYL | Xylose |
| GLU | Glukose |
| RIB | Ribose |
| Zuckertypen nach Lechevalier et al. 1971 | |
| Fettsäuretypen nach Kroppenstedt 1985 und 1992. | |
| 9/4 | MK-9 (H₄) |
| 9/6 | MK-9 (H₆) |
| 9/8 | MK-9 (H₈) |
| MS | Mycolsäuren |
| PE | Phosphatidylethanolamin |
| OH-PE | Hydroxy-PE |
| met PE | Phosphatidimethylethanolamin |
| PC | Phosphatidylcholin |
| NPG | Phosphatidylglucosamin |
| PI | Phosphatidylinositol |
| PIM | Phosphatidylinositolmannosid |
| PG | Phosphatidylglycerol |
| DPG | Diphosphatidylglycerol |
| GL | Glykolipide |
| Fettsäuren iso-16 | iso-Hexadecansäuren oder |
| | 14Methyl-Pentadecansäuren |
| 10-Me-18 | Tuberkulostearinsäure |
| 2-OH-16 | 2-Hydroxy-palmitinsäure |

### Identitifierzung von GF270 und GF376

Die Identifizierung dieser Stämme basierte auf 5 voneinander unabhängigen Eigenschaften.
1. Morphologie und Farbe der Kolonien: kurz verzweigte Hyphen, die in Stäbchen- und Sporen-ähnliche Elemente desintegrieren. Die Kolonien von GF270 und GF376 sind lachsfarben rot (RAL 3022).
2. Diaminosäuren des Peptidoglycans: meso-Diaminopimelinsäure
3. Mycolsäuren: Rhodococcus Mycolsäuren: Die Bestimmung der langkettigen Mycolsäure wurde mittels Hochtemperatur-Gaschromatographie durchgeführt. Die Elutionsprofile der Mycolsäuren von GF270 und GF376 waren identisch. Die Mycolsäurenlänge für GF270 und GF376 war C₃₈-C₄₆. Die Mycolsäuremuster wurden mit Mycolsäuremuster von Rhodococcus-Stämmen verglichen. GF270 wurde mit einem sehr niedrigen Korrelationsfaktor (0,086) zu Rhodococcus rhodochrous gehörend identifiziert; GF376 konnte nicht durch diese Methode identifiziert werden.
4. Fettsäuremuster: unverzweigte, gesättigte und nicht gesättigte Fettsäuren inklusive Tuberculostearinsäure. Das Fettsäuremuster ist diagnostisch für alle Rhodococcus-Gattungen und nahen Verwandten Mycobacterium, Nocardia, Dietzia, Tsukamurella und einigen Corynebakterien-Spezies. Die Identifizierung auf der Spezies-Ebene wurde erhalten durch qualitative und quantitative Unterschiede im Fettsäuremuster von GF270 und GF376 mit den Fettsäuremustern von Rhodococcus-Spezien.
5. Die 16S r DNA Teilsequenzen von GF270 und GF376 waren identisch (100%), obwohl der Vergleich von ihnen zu den Rhodococcus-Stämmen nur 99,1%-ige Ähnlichkeit zum nächst verwandten Rhodococcus rhodochrous aufwies.

Basierend auf den chemotaxischen und molekularbiologischen Ergebnissen kann man daraus schliessen, dass GF270 und GF376 Stämme einer neuen Rhodococcus-Spezies sind. GF270 und GF376 sind nah verwandt zu Rhodococcus rhodochrous in ihrer 16S r DNA (99,1%).

Der Enzymextrakt kann durch fachmännisch übliches Aufschliessen der Mikroorganismen gewonnen werden, wie beispielsweise durch Aufschliessen mittels Ultraschall, mittels der French-Press- oder der Lysozym-Methode. Dieses Enzymextrakt sowie natürlich auch die ganzen Mikroorganismenzellen können für die Durchführung des Verfahrens an einem geeigneten Trägermaterial, üblicherweise eingebettet in einem Polymer, immobilisiert, oder auf einem geeigneten Trägermaterial absorbiert werden.

Die erfindungsgemässen Enzyme mit Nitrilhydratase-Aktivität sind erhältlich aus den Mikroorganismen der Gattung Amycolatopsis und befähigt, ein Nitril in ein Amid umzusetzen, insbesondere sind sie erhältlich aus Amycolatopsis NA40 (DSMZ 11617). Diese Enzyme weisen insbesondere folgende Eigenschaften auf:
a) ein pH-Optimum von pH 6,5 ± 1,0;
b) ein Temperatur-Optimum zwischen 35 und 40 °C bei einem pH von 7,0;
c) einen K_{M}-Wert für das Substrat 3-Cyanpyridin von 41,7 mM ± 7,7 mM (20 °C, 45 mM Phosphat-Puffer, pH 7,0).

Insbesondere besitzen die Enzyme
d) ein Molekulargewicht von 106 kDa, wie beispielsweise bestimmt durch SDS-PAGE.

Als Substrate für die Biotransformation können generell Nitrile eingesetzt werden. Zweckmässig werden entweder aliphatische Nitrile mit 1 bis 10 Kohlenstoffatomen, ggf. substituiert mit beispielsweise Hydroxy, Amino, Halogen oder Carboxy, oder substituierte oder unsubstituierte aromatische Nitrile mit 4 bis 10 Kohlenstoffatomen im aromatischen Ringsystem eingesetzt. Als aliphatische Nitrile mit 1 bis 10 Kohlenstoffatomen können Dinitrile, Hydroxynitrile, Aminonitrile wie z.B. n-Octannitril, Cyanessigsäure, Isocapronitril, n-Valeronitril, Adiponitril, Glutaronitril, Succinonitril, Sebacinsäuredinitril, Propionitril, Crotonsäurenitril, Acrylnitril, Methacrylnitril, n-Buttersäurenitril oder Azelainsäurenitril verwendet werden. Als aromatische Nitrile mit 4 bis 10 Kohlenstoffatomen können Nitrile der allgemeinen Formeln 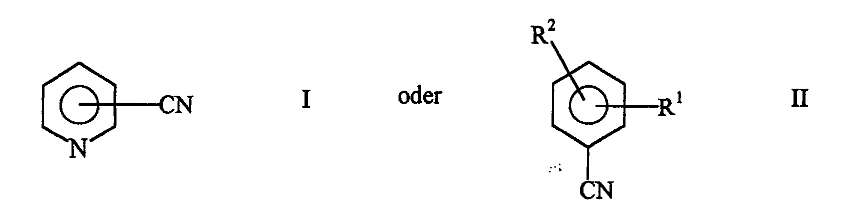 worin R¹ und R² ein Wasserstoffatom, ein Halogenatom oder C₁₋₄-Alkyl bedeuten, verwendet werden. Als Halogenatom kann F, Cl, Br oder J verwendet werden.

Als C₁₋₄-Alkyl kann Methyl, Ethyl, Propyl, Isopropyl, Butyl, Isobutyl oder tert-Butyl verwendet werden. Zweckmässige Vertreter der aromatischen Nitrile der allgemeinen Formeln I oder II sind 2-, 3- oder 4-Cyanpyridin, Benzonitril, Fluor-, Chlor-, Brombenzonitril, wie z. B. o-, m-, p-Chlorbenzonitril oder 2-Chlor-3-cyanpyridin. Vorzugsweise wird als aromatisches Nitril mit 4 bis 10 Kohlenstoffatomen 3-Cyanpyridin verwendet.

Zweckmässig wird die Biotransformation unter einmaliger oder kontinuierlicher Substratzugabe so durchgeführt, dass die Substratkonzentration 40 Gew.%, vorzugsweise 30 Gew.% nicht übersteigt.

Zweckmässig wird das Verfahren mit ruhenden (nicht wachsende) Zellen durchgeführt.

Als Medien für die Biotransformation sind die in der Fachwelt üblichen geeignet, wie beispielsweise niedermolare Phosphatpuffer, HEPES-Puffer, Citrat-Puffer, Borat-Puffer, die Medien gemäss Tabelle 1 bis 3 bzw. abgewandelte Formen davon wie z.B. die in Beispiel 8 (1) beschriebenen, oder TRIS/HCl-Puffer.

Zweckmässig wird die Biotransformation bei einer Temperatur von 0 bis 50 °C und bei einem pH-Wert zwischen pH 4,5 und pH 10, vorzugsweise bei einer Temperatur von 20 bis 40 °C und bei einem pH-Wert zwischen pH 4,5 und pH 10,0, durchgeführt.

In einer besonders bevorzugten Ausführungsform kann die Biotransformation in Gegenwart von C₁₋₄-Alkoholen durchgeführt werden. Als C₁₋₄-Alkohole können Methanol, Ethanol, Propanol oder Butanol eingesetzt werden. Vorzugsweise wird Methanol verwendet.

Nach der Umsetzung können dann die entsprechenden Amide durch übliche Aufarbeitungsmethoden wie beispielsweise durch Kristallisation isoliert werden.

### Beispiele:

### Beispiel 1

### Anzucht von Mikroorganismen der Gattung Actinomadura und Amycolatopsis

**a)** Verschiedene Bodenproben wurden im Anreicherungsmedium gemäss Tabelle 1 mit verschiedenen Nitrilen oder Amiden als C- und N-Quelle angeimpft und 7 - 10 Tage bei 37°C oder 45°C bebrütet. Danach wurden die Kulturen in dem gleichen Medium überimpft und nochmals 7-10 Tage bei 37°C kultiviert. Das Ganze wurde 3mal wiederholt. Anschliessend wurden die Kulturen verdünnt und ausplattiert, um einzelne Kolonien zu erhalten. Die Platten wurden 5 Tage bei 37°C bebrütet. Dann wurden die unterschiedlichen Kolonien für die gewünschte Aktivität getestet.
Auf diese Weise wurden Amycolatopsis NA40 (DSMZ 11617) und Amycolatopsis NE31 (DSMZ 11616) isoliert und dann im optimierten Medium (Tabelle 3) 90 - 100 h unter Schütteln bei 37 °C angezogen.
Für Amycolatopsis NE31 (DSMZ 11616), Actinomadura spadix E3733 und Actinomadura spadix E3736 diente als C- und N-Quelle Adiponitril, für Amycolatopsis NA40 (DSMZ 11617), Actinomadura spadix 45A32 diente als C- und N-Quelle Azelanitril, für Actinomadura spadix 4501 diente n-Octanitril als C- und N-Quelle und für Actinomadura spadix C15 diente Cyanessigsäure als C- und N-Quelle.
**b)** Amycolatopsis NA40 wurde in dem Medium gemäss Tabelle 3 kultiviert. Die Kultivierung wurde in Subkulturen (4 ml/Röhrchen) und in einer "Hauptkultur" (500 ml/Kolben) unter aerobischen Bedingungen bei einer Temperatur von 37°C 2 bzw. 3 bis 4 Tage durchgeführt. Das Zellwachstum wurde turbimetrisch bei 610 nm gemessen und das Trockengwicht der Zellen folgendermassen berechnet: Gewicht der trockenen Zellen in mg/ml = OD_{610 nm} x 0,277.

**Tabelle 1**

| **Anreicherungsmedium** | |
|---|---|
| Nitril | 2,0 g |
| KH₂PO₄ | 7,0 g |
| MgSO₄·7H₂O | 0,1 g |
| Vitaminmischung | 1,0 ml |
| CoCl₂·6H₂O | 2,0 mg |
| FeSO₄·7H₂O | 2,0 mg |
| Mit Wasser auf 1l auffüllen (pH 6,7 - 7,3) | |

**Tabelle 2**

| **Basalmedium** | |
|---|---|
| Maltose | 2,0 g |
| NaNO₃ | 1,0 g |
| K₂HPO₄ | 0,1 g |
| MgSO₄ · 7H₂O | 0,05 g |
| Mit Wasser auf 100 ml auffüllen (pH 7,0) | |

**Tabelle 3**

| **Optimiertes Medium** | |
|---|---|
| D-Glucose | 4,5 g |
| Fleischextrakt | 0,5 g |
| K₂HPO₄ | 0,1 g |
| MgSO₄·7H₂O | 0,05 g |
| CoCl₂·6H₂O | 1,0 mg |
| Mit Wasser auf 100 ml auffüllen (pH 7,0) | |

### Beispiel 2

### Biotransformationen mit Mikroorganismen der Gattung Actinomadura und Amycolatopsis

(1) Zur Bestimmung der Nitrilhydratase-Aktivität wurde eine Reaktionsmischung (2 ml) mit 3-Cyanpyridin (1,0 M, 1,0 ml), Kaliumphosphatpuffer (pH 7,0, 0,1 M, 0,5 ml) und 0,5 ml Zellsuspension unter Rühren bei 20 °C für 30 min. inkubiert. Die Reaktion wurde durch Zugabe von 0,2 ml 3 N HCl gestoppt. Nach kurzem Zentrifugieren wurde das gebildete Nicotinamid mittels HPLC (Shimadzu SPD 6A System mit einer C18-Säule (Develosil ODS-HG-5, 4,6 x 250 cm); Laufmittel: 10 mM KH₂PO₄/H₃PO₄ (pH 2,8)/Acetonitril 9:1 (v/v); Durchflussrate: 1 ml/min; die Absorption wurde bei 230 nm gemessen) bestimmt. Die spezifische Aktivität wurde ausgedrückt als µmol gebildetes Nikotinamid / ml / min. /-OD₆₁₀ₙₘ.
Die Umsetzungsraten von aliphatischen Nitrilen im Anreicherungsmedium (Tabelle 1) mit isolierten Bakterien sind in Tabelle 5, der Einfluss von Induktoren und Cofaktoren im Basalmedium (Tabelle 2) ist in Tabelle 4 und der Aktivitätsvergleich von Amycolatopsis zu Rhodococcus im Basalmedium (Tabelle 2) ist in Tabelle 6 zusammengefasst. Die Ergebnisse aus Tabelle 4 zeigen, dass die Nitrilhydratase aus Amycolatopsis NA40 konstitutiv exprimiert wird, aber der Cofaktor Cobalt für die Aktivität notwendig ist.
(2) **Effekt der Temperatur auf das Wachstum von NA40** Subkulturen (2 ml) wurden im Medium gemäss Tabelle 3 für 2 Tage bei 37 °C inkubiert, die dann in Schüttelkolben mit 20 ml Medium gemäss Tabelle 3 überimpft wurden. Die Kultivierung wurde bei 37, 40, 45, 50 und 55 °C für 3 bis 4 Tage unter Schütteln durchgeführt. Das Zellwachstum wurde gemessen und die Nitrilhydratase-Aktivität wurde bei 20 °C ermittelt. Tabelle 7 zeigt den Einfluss der Temperatur auf die Nitilhydratase-Aktivität und auf das Zellwachstum.

**Tabelle 4**

| **Einfluss von Induktoren und Cofaktoren auf die spezifische Aktivität im Basalmedium** | | | |
|---|---|---|---|
| **Induktor** | **Wachstum (OD610 nm)** | **Gesamtaktivität (µ mol/ml/min)** | **Spezif. Aktivität (µ mol/ml/min/OD)** |
| - | 1,26 | 20,9 | 16,6 |
| ε-Caprolactam | 0,66 | 9,52 | 14,5 |
| Crotonamid | 3,41 | 22,9 | 6,72 |
| Methacrylamid | 3,33 | 2,46 | 0,74 |
| Butyramid | 2,19 | 0,19 | 0,88 |
| Propionamid | 1,91 | 0,92 | 0,48 |
| Harnstoff | 1,72 | 2,97 | 1,73 |
| | | | |

| **Cofactor** | **Wachstum (OD610 nm)** | **Gesamtaktivität (µ mol/ml/min)** | **Spezif. Aktivität (µ mol/ml/min/OD)** |
|---|---|---|---|
| - | 7,97 | 0,10 | 0,01 |
| FeSO₄·7H₂O | 8,32 | 3,36 | 0,40 |
| CoCl₂·6H₂O | 8,41 | 47,8 | 5,68 |

**Tabelle 5**

| **Umsetzungsraten von aliphatischen Nitriten mit den isolierten Bakterien** | | | | | |
|---|---|---|---|---|---|
| | **Stämme** | **Substrate** | **Wachstum (OD610nm)** | **Gesamtakti- vität (µmol/ ml/min)** | **Spez. Ak- tivität (µmol/ml/ min/OD)** |
| Amycolatopsis | NE31 (DSMZ 11616) | Adiponitril | 2,68 | 0,377 | 0,141 |
| Actinomadura spadix | E3733 | Adiponitril | 1,62 | 0,347 | 0,214 |
| " | E3736 | Adiponitril | 1,36 | 3,00 | 2,21 |
| " | 45A32 | Azelanitril | 5,81 | 18,8 | 3,23 |
| " | 4501 | n-Octannitri | 7,24 | 32,2 | 4,45 |
| " | C15 | Cyanessig- | 2,04 | 7,01 | 3,43 |
| | | säure | | | |
| Amycolatopsis | NA40 (DSMZ 11617) | Azelanitril | 5,92 | 33,0 | 5,57 |

**Tabelle 6**

| **Aktivität von Amycolatopsis im Vergleich zu Rhodococcus rhodochrous J1** | | |
|---|---|---|
| | **Mikroorganismus Amycolatopsis NA40 (DSMZ 11617) (µ mol/ml/min)** | **Mikroorganismus Rhodococcus rhodochrous J1** |
| Aktiviät für 3-Cyanpyridin | 303 | 314 |

| | **gereinigtes Enzym aus NA40 (µmol/min./mg Pro- tein)** | **gereinigtes Enzym aus J1 (µmol/min./mg Protein)** |
|---|---|---|
| Aktivität für 3-Cyanpyridin | 1110 | 371 |

(3) Zur Bestimmung der Aktivität von NA40 bez. mehreren Substraten wurden Zellen mit einem Trockengewicht von 0,0388 mg in dem oben beschriebenen Puffer inkubiert. Die Reaktion wurde durch Zugabe des entsprechenden Substrats gestartet und 10 min unter Schütteln bei 20 °C inkubiert. Die Reaktion wurde durch Zugabe von 0,2 ml 2 N HCl gestoppt und die Reaktionsmischung kurz zentrifugiert. Der Überstand wurde mittels HPLC oder Gaschromatographie analysiert. Tabelle 8 zeigt die Testbedingungen zur Substratspezifität und Tabelle 9 zeigt die Substratspezifität von ruhenden NA40 Zellen für verschiedene Substrate.
Die jeweiligen Testbedingungen sind in Tabelle 8 und die Ergebnisse in Tabelle 9 zusammengefasst.

**Tabelle 7**

| **Einfluss der Wachstumstemperatur auf die Nitrilhydratase-Aktivität und auf das Zellwachstum** | | | | |
|---|---|---|---|---|
| **Temperatur** | **Wachstum (mg/ml)** | **Gesamtaktivität (µmol/ml/min.)** | **Spez. Aktivität (µmol/ml/min./mg)** | **Relative Aktivität (%)** |
| 37 °C | 6,16 | 4,69 | 0,761 | 100 |
| 40 °C | 5,79 | 9,89 | 1,71 | 225 |
| 45 °C | 6,56 | 4,83 | 0,736 | 97 |
| 50 °C | 5,96 | 1,16 | 0,195 | 26 |

**Tabelle 8**

| **Testbedingungen zur Substratspezifität** | | | |
|---|---|---|---|
| **Substrat** | **Substrat (mM)** | **Analyse- methode** | **gebildetes Amid** |
| 3-Cyanpyridin | 1,0 | HPLC | Nicotinamid |
| 2-Cyanpyridin | 0,25 | HPLC | 2-Picolinamid |
| 4-Cyanpyridin | 0,25 | HPLC | Pyridin-4-carboxamid |
| Crotonitril | 0,4 | HPLC | Crotonamid |
| Benzonitril | 0,03 | HPLC | Benzamid |
| Acrylonitril | 0,4 | HPLC | Acrylamid |
| o-Chlorbenzonitril | 0,15 | HPLC | o-Chlorbenzamid |
| m-Chlorbenzonitril | 0,15 | HPLC | m-Chlorbenzamid |
| p-Chlorbenzonitril | 0,15 | HPLC | p-Chlorbenzamid |
| 2-Chlor-3-cyanpyridin | 0,15 | HPLC | 2-Chlornikotinamid |
| Acetonitril | 0,4 | GC | Acetamid |
| Propionitril | 0,4 | GC | Propionamid |
| Methacrylnitril | 0,4 | GC | Methacrylamid |
| n-Butyronitril | 0,4 | GC | n-Butyramid |
| o-, m-, p-Chlorbenzonitril und 2-Chlor-3-cyanpyridin wurden in Methanol gelöst zur Reaktionsmischung gegeben. | | | |

**Tabelle 9**

| **Substratspezifität von NA40 Nitrilhydratase** | | | | |
|---|---|---|---|---|
| **Substrat** | **relative Aktivität (%)** | | **Substrat** | **relative Aiktivität (%)** |
| 3-Cyanpyridin | 100 | | m-Chlorbenzonitril | 75 |
| 4-Cyanpyridin | 168 | | p-Chlorbenzonitril | 16 |
| 2-Cyanpyridin | 128 | | 2-Chlor-3-cyanpyridin | 126 |
| Benzonitril | 51 | | Acetonitril | - |
| Crotonitril | 52 | | Propionitril | 105 |
| Acrylnitril | 115 | | Methacrylnitril | 130 |
| o-Chlorbenzonitril | 96 | | n-Butyronitril | 194 |

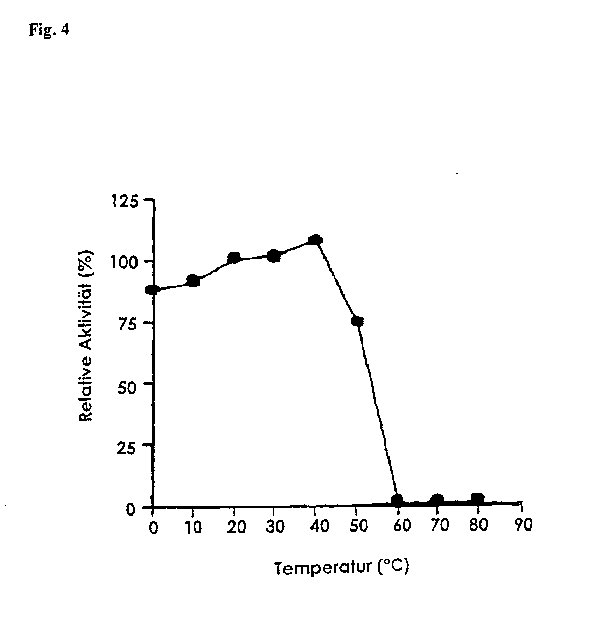

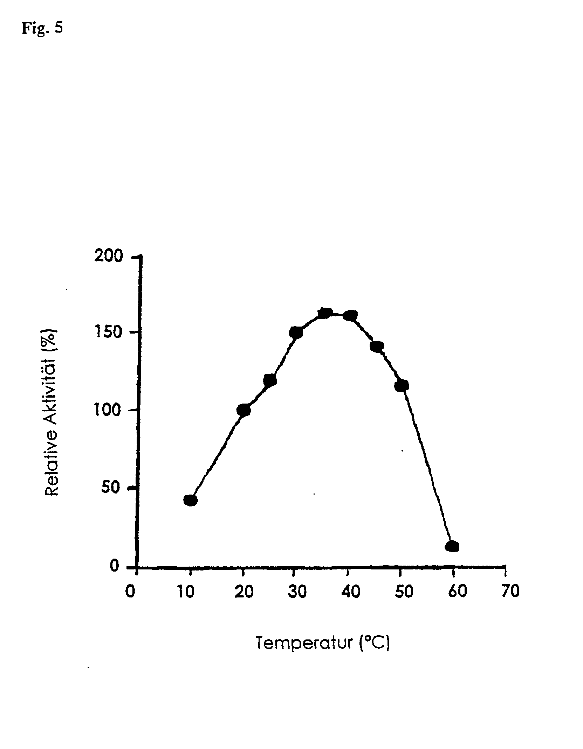

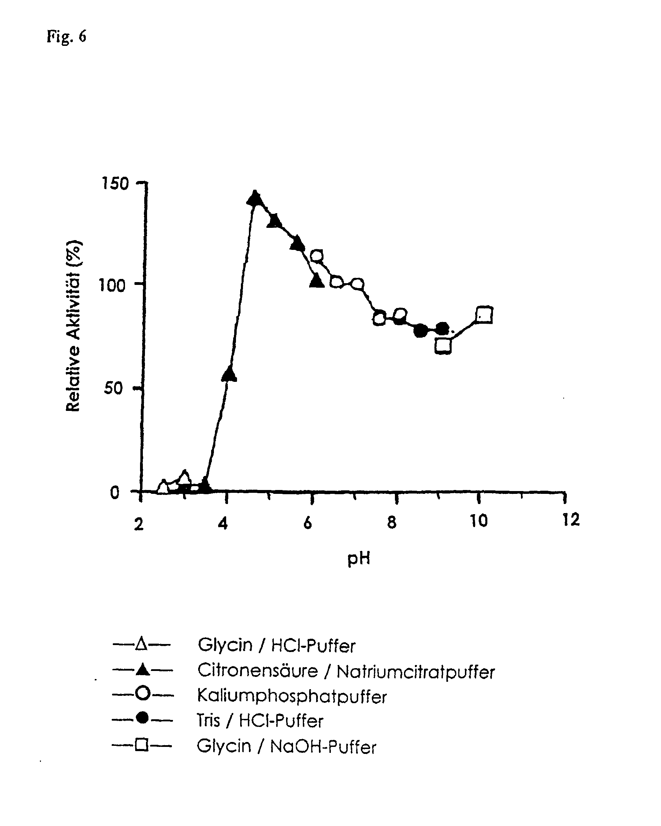

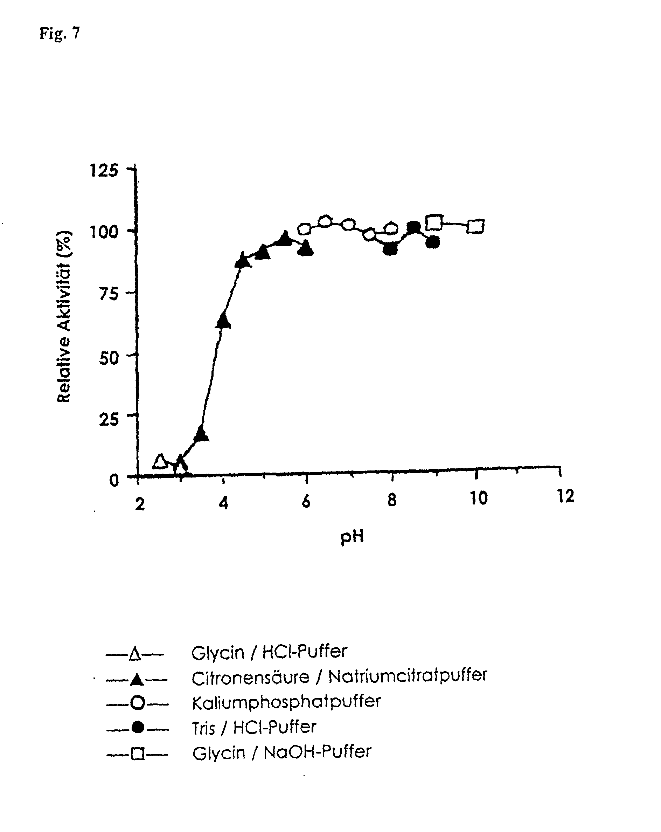

(4) **Temperaturoptimum und thermische Stabilität in ruhenden Zellen**
Die Reaktion wurde 10 min. lang in der Standardreaktionsmischung durchgeführt. Das Temperaturoptimum lag zwischen 35 und 40°C (Fig. 5). Danach wurden die Zellen 30 min. lang bei verschiedenen Temperaturen inkubiert und die Aktivität unter Standardreaktionsbedingungen getestet. Wie aus Fig. 4 ersichtlich, lag die Hitzestabilität ca. bei 40°C.
(5) **pH Optimum und pH-Stabilität in ruhendes Zellen**
Hierfür wurde die Reaktion 10 min. lang in der Standardreaktionsmischung, in der der Kaliumphosphatpuffer durch verschiedene 0,1 M Puffer ausgetauscht worden war, durchgeführt. Wie aus Fig. 6 ersichtlich, lag das pH-Optimum zwischen 4,5 und 10. Nachdem die Zellsuspension bei verschiedenen pH-Werten bei 20°C 30 min. lang inkubiert worden waren, wurden die Zellen zentrifugiert. Dann wurden die Zellen gewaschen und in 0,1 M Kaliumphosphatpuffer, pH 7,0 resuspendiert. Die Reaktion wurde 10 min. lang durch Zugabe von 3-Cyanpyridin unter Standardbedingungen durchgeführt. Das Enzym war stabil zwischen pH 4,5 und pH 10,0 (Fig. 7).
6) **Akkumulation von Nikotinamid aus 3-Cyanpyridin mittels NA40**
Die Reaktion wurde in einer Reaktionsmischung (30 ml), enthaltend 500 mM 3-Cyanpyridin, 40 mM Kaliumphosphatpuffer (pH 7,0) und ruhende Zellen (Trockengewicht 2,3 mg) durchgeführt. Während der Reaktion wurde 3-Cyanpyridin (500 mM) 7mal hinzugegeben, sobald dieses verbraucht war. Innerhalb 15 h wurden auf diese Weise 4,0 M 3-Cyanpyridin hinzugegeben und 3,89 M (475 g/l) Nikotinamid gebildet, entsprechend einer Ausbeute von 97,3 %. Nikotinsäure wurde nicht gebildet.

### Beispiel 3

### Identifizierung von Mikroorgansimen der Gattungen Amycolatopsis

Folgende 5 chemotaxonomische Marker stützten die Identifizierung:
1. Diagnostische Aminosäure des Peptidoglycans: meso-Diaminopimelinsäure
2. Diagnostische Zucker: Arabinose und Galaktose
3. Mycolsäuren: Mycolsäuren fehlen
4. Menachinone: MK-9 (H₄)
5. Fettsäuremuster: Iso/Anteiso-verzweigte und 2 Hydroxy-Fettsäuren, geringe Mengen an 10-methylverzweigten Fettsäuren wurden zusätzlich nachgewiesen. Dieses Fettsäuremuster wurde bei allen Vertretern der Gattung Amycolatopsis gefunden (Fettsäuremuster 3f)

Die Kombination dieser chemischen Merkmale ist diagnostisch für alle Spezies der Gattung Amycolatopsis.

Die Fettsäuredaten der beiden Kulturen wurden mit Hilfe von Hauptkomponenten-Analysen mit den Einträgen der Fettsäuredatenbank verglichen. Mit dieser Methode konnte sowohl NE31 als auch NA40 der Gattung Amycolatopsis zugeordnet werden, eine Identifizierung zur Spezies war jedoch nicht möglich, da der Korrelationsfaktor zu niedrig war. Der Vergleich der Fettsäuremuster beider Stämme zeigte aber auch, dass es sich um zwei Stämme verschiedener Arten handelt.

Das Ergebnis wurde durch die Resultate der 16S rDNA Sequenz Analyse bestätigt. Auch hier erfolgte eine Zuordnung zur Gattung Amycolatopsis, jedoch zu keiner der beschriebenen Amycolatopsis Spezies. Bei dieser Methode wurde die Sequenz der 16S rDNA durch die direkte Sequenzierung des PCR amplifizierten 16S rDNA Gens bestimmt. Der diagnostische Teil der 16S rDNA Sequenz wurde mit den Sequenzen der Typus Arten der Gattung Amycolatopsis und verwandter Taxa verglichen. Das Ergebnis zeigte, dass der Stamm der Gattung Amycolatopsis angehört. Die höchste Übereinstimmung wurde mit 96,9 % (NA40) und 96,1 % (NE31) zu Amycolatopsis methanolica gefunden. Untereinander wiesen die beiden Isolate eine Übereinstimmung in den Sequenzen von 99,0 % auf. Unsere Untersuchungen an Vertretern der Gattung Amycolatopsis haben gezeigt, dass für eine gute Spezies-Identifizierung der Korrelationsfaktor höher als 99,5 % sein musste. Da der Wert mit 96,9 % deutlich unter 99,5 % liegt, kann man davon ausgehen, dass es sich bei den beiden Isolaten um keine Vertreter von bekannten Amycolatopsis Spezies handelte.

Aufgrund vorliegender Resultate konnten die Isolate keiner der bekannten Amycolatopsis-Arten zugeordnet werden. Wir gingen davon aus, dass es sich bei NA40 und NE31 um Stämme von zwei neuen, bisher nicht beschriebenen Spezies der Gattung Amycolatopsis handelte.

### Identifizierungscharacteristika von Mikroorganismen der Gattung Amycolatopsis

| Farbe Luft-Myzel | |
|---|---|
| Farbe Substrat-Myzel | |
| Farbe diffund. Pigment | |
| Zuckerspektrum | |
| ARA | + |
| GAL | + |
| MAD | - |
| XYL | - |
| GLU | v |
| RIB | + |
| Typ | A |
| DAP | DL |

| Menachinone (in %) | |
|---|---|
| 8/4 | - |
| 9/0 | (+) |
| 9/2 | + |
| 9/4 | +++ |
| 9/6 | - |
| 9/8 | - |
| 16S rDNA Homologie | >99,5 % |

| Phospholipide | |
|---|---|
| PE | + |
| OH-PE | + |
| lyso PE | - |
| met PE | - |
| PC | - |
| NPG | - |
| PI | + |
| PIM | v |
| PG | + |
| DPG | + |
| GL | - |
| Typ | II+OH-PE |

| Fettsäuren | |
|---|---|
| iso 16 | +++ |
| iso 15 | + |
| iso 17 | (+) |
| anteiso 15 | + |
| anteiso 17 | (+) |
| 10-Me 16 | (+) |
| 10-Me 17 | + |
| 2-OH 15 | + |
| 2-OH 16 | + |
| Typ | 3f |
| MS | - |

### Beispiel 4

### Reinigung der Nitrilhydratase aus Mikroorganismenstamm NA40.

Der Stamm wurde für 3 Tage im Medium gemäss Tabelle 3 bei 37 °C kultiviert. Die Zellen einer 2-1-Kultur wurden mittels Zentrifugation geerntet und dann in 0,85 %iger NaCl-Lösung resuspendiert. Dann wurden die Zellen in 0,1 M Kaliumphosphatpuffer (pH 7,0) enthaltend 44 mM n-Buttersäure überführt und mit Ultraschall behandelt. Das Zellextrakt wurde zentrifugiert und die Zelltrümmer entfernt. Dieses Extrakt wurde zur Enzymreinigung verwendet. Während der gesamten Reinigung wurde Kaliumphosphatpuffer (pH 7,0) enthaltend 44 mM n-Buttersäure verwendet. Wie aus Tabelle 10 ersichtlich, wurde das Enzym in 3 Schritten bis zur Homogenität gereinigt.

**Tabelle 10**

| **Reinigung der Nitrilhydratase aus NA40** | | | | |
|---|---|---|---|---|
| | **Total Aktivität (Units)** | **Total Protein (mg)** | **Spez. Aktivität (U/mg)** | **Anreicherung** |
| Zellfreies Extrakt | 73300 | 1020 | 71,9 | 1 |
| DEAE-Sephacel | 68000 | 110 | 620 | 8,62 |
| Phenyl-TOYOPEARL | 64800 | 61,4 | 1105 | 15,4 |
| 1 Unit: Die Enzymmenge, die die Bildung von 1 µmol Nikotinamid/min bei 20 °C katalysiert. | | | | |

### Beispiel 5

### Characterisierung der Nitrilhydratase

### (1) Bestimmung des Molekulargewichts, der Untereinheitenstruktur und des Cobaltionenen-Gehaltes

Das Molekulargewicht wurde zu 106 kDa bestimmt durch Chromatographie an TSK-Gel-Säule G3000 SW (0,75 x 60 cm) unter Verwendung eines 0,1 M Kaliumphosphatpuffers (pH 7,0) mit 0,2 M KCl und 44 mM n-Buttersäure. Es wurde ermittelt, dass das Enzym aus 2 verschiedenen Untereinheiten α und β besteht, deren Molekulargewicht jeweils zu 30000 und 26000 bestimmt wurde.

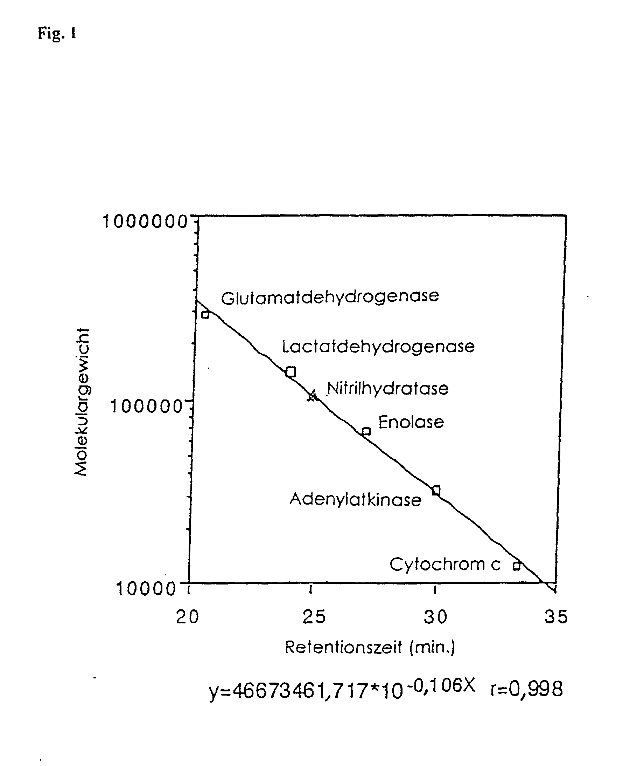

Fig. 1 zeigt die Bestimmung des Molekulargewichts durch Chromatographie an TSK-Gel G3000 SW.

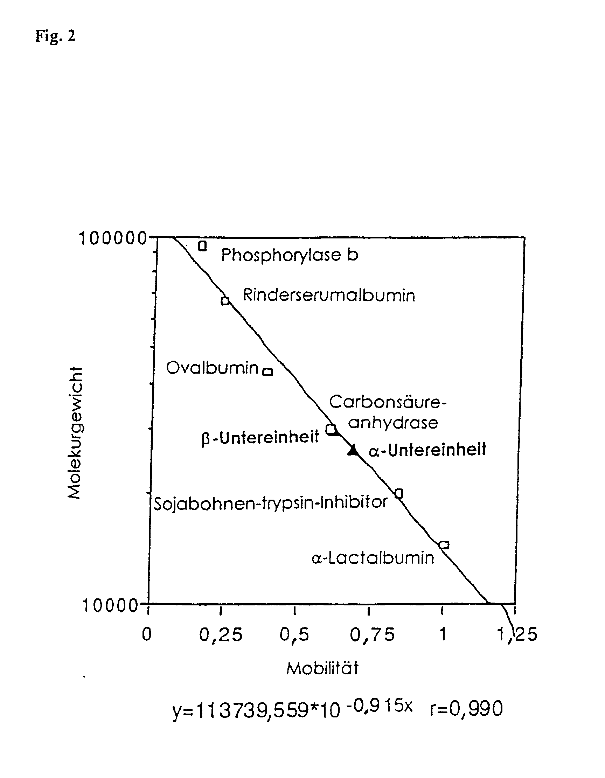

Fig. 2 zeigt die Bestimmung des Molekulargewichts mittels SDS-PAGE

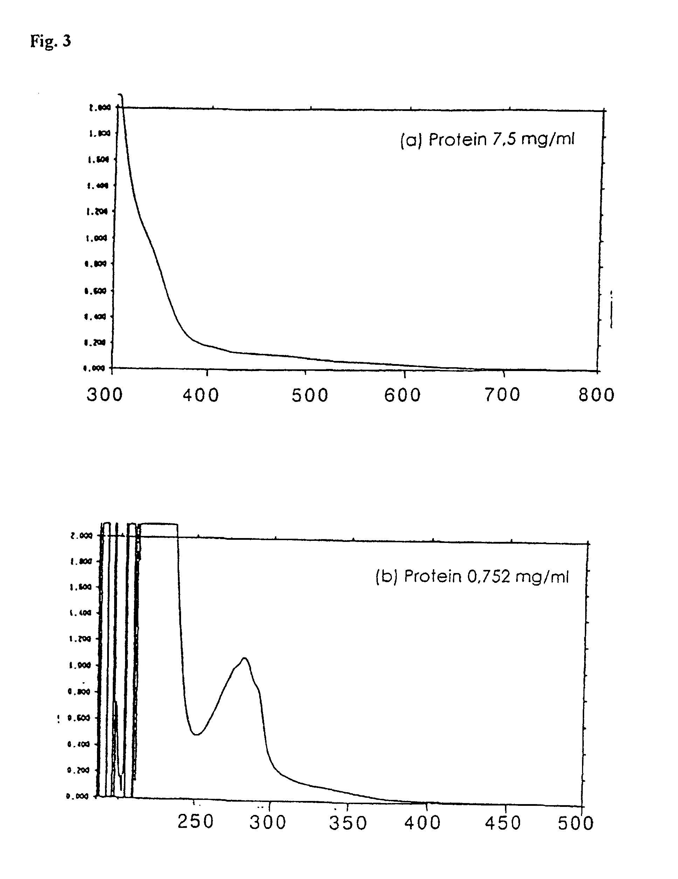

Fig. 3 zeigt das Absorptionsspektrum des gereinigten Enzyms. Eine breite Absorption von 300 - 400 nm wurde beobachtet.

### (2) Substratspezifität des gereinigten Enzyms

Die Bestimmung der Substratspezifität wurde in analoger Weise wie in Beispiel 2 (1) bestimmt. Die Ergebnisse sind in Tabelle 11 zusammengefasst.

**Tabelle 11**

| **Substratspezifität der gereinigten Nitrilhydratase** | | | | |
|---|---|---|---|---|
| Substrate | (M) | Gesamtaktivität | relative Aktivität (%) | |
| | | (µmol/ml/min.) | Enzymreaktion | Reaktion mit ruhenden Zellen |
| 3-Cyanpyridin | 1,0 | 17,7 | 100 | 100 |
| 2-Cyanpyridin | 0,25 | 39,1 | 221 | 128 |
| 4-Cyanpyridin | 0,25 | 31,6 | 179 | 168 |
| Crotonitril | 0,4 | 11,9 | 67 | 52 |
| Benzonitril | 0,03 | 11,3 | 64 | 51 |
| Acrylnitril | 0,4 | 16,6 | 94 | 115 |
| o-Chlorbenzonitril | 0,15 | 22,4 | 127 | 96 |
| m-Chlorbenzonitril | 0,15 | 15,9 | 90 | 75 |
| p-Chlorbenzonitril | 0,15 | 2,30 | 13 | 16 |
| 2-Chlor-3-cyanpyridin | 0,15 | 16,0 | 90 | 126 |
| Acetonitril | 0,4 | - | - | - |
| Propionitril | 0,4 | 39,3 | 222 | 105 |
| Methacrylnitril | 0,4 | 22,1 | 125 | 130 |
| n-Butyronitril | 0,4 | 17,9 | 101 | 194 |
| 1,7 Units Enzym wurden zur Reaktionsmischung (2,0 ml) hinzgegeben. Die Reaktionsmischung enthielt das jeweilige Substrat in 45 mM Phosphatpuffer (pH 7,0). | | | | |

### (3) Bestimmung des K_{M}-Wertes

Der K_{M}-Wert wurde anhand des Lineweaver-Burk Diagramms für 3-Cyanpyridin zu 41,7 mM und für Acrylnitril zu 3,7 mM bestimmt. Verglichen mit Rhodococcus rhodochrous J1, der einen K_{M}-Wert bez. 3-Cyanpyridin von 200 mM aufweist ist der von NA40 wesentlich geringer. Dies ist eines der Hauptvorteile von NA 40.

### (4) Hitzestabilität und Temperaturoptimum

Das gereinigte Enzym wurde für 30 min. bei pH 7,0 bei unterschiedlichen Temperatuten inkubiert und dann wurde die Umsetzung von 3-Cyanpyridin zu Nikotinsäureamid für 1 min. bei 20 °C gemessen. Das Enzym wurde bei einer Temperatur von grösser als 40 °C inaktiviert. Die Hitzestabilität lag wie bei ruhenden Zellen bei ca. 40 °C, das Temperaturoptimum zwischen 35 und 40 °C (Fig. 5).

### (5) pH-Optimum und pH-Stabilität

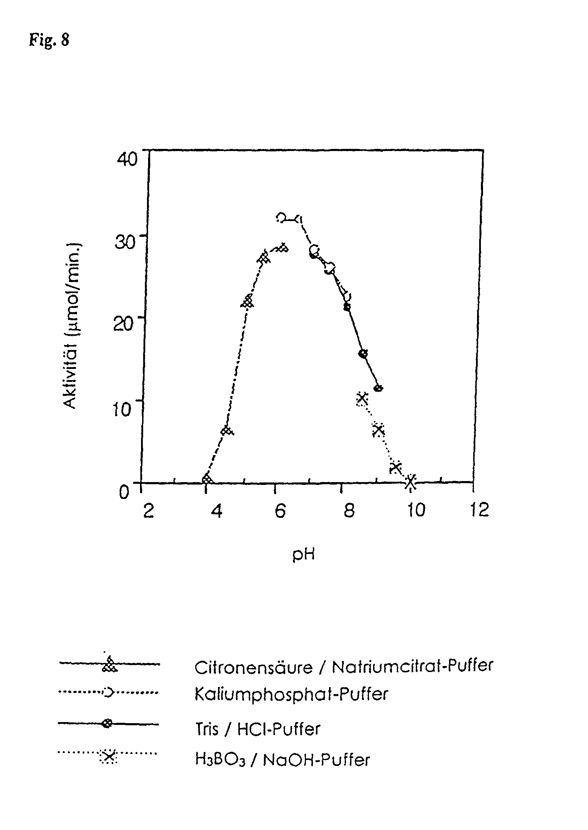

Hierfür wurde die Reaktion von 3-Cyanpyridin zu Nikotinsäureamid bei 20 °C in einer Reaktionsmischung (2,0 ml), enthaltend verschiedene Puffer (42,5 mM), 1,71 Units gereinigtes Enzym und 500 mM 3-Cyanpyridin, durchgeführt. Das pH-Optimum lag ca. bei pH 6,5 ± 1,0 (Fig. 8).

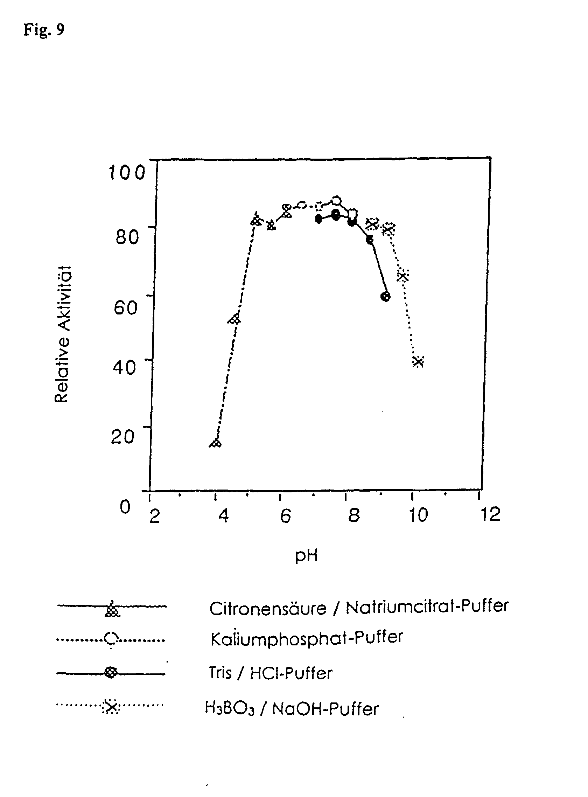

Zur Bestimmung der pH-Stabilität wurden 4,2 Units gereinigtes Enzym in verschiedenen Puffern (45 mM) bei 20 °C für 1 h inkubiert. Ein Teil der inkubierten Lösung 1,71 Units wurden zur Standardreaktionsmischung (vgl. Beispiel 2(1)) gegeben. Die verbliebene Aktivität wurde bestimmt. Das Enzym war in einem pH-Bereich von pH 5 - 9 stabil. Das Ergebnis ist in Fig. 9 dargestellt.

### (6) Inhibitoren

Der Effekt von verschiedenen Inhibitoren wurde bestimmt. Die Ergebnisse sind in Tabelle 12 zusammengefasst.

**Tabelle 12**

| **Effekt verschiedener Inhibitoren auf das gereinigte Enzym** | | |
|---|---|---|
| **Inhibitor** | **mM** | **relative Aktivität (%)** |
| - | | 100 |
| N-Ethylmaleinimid | 1 | 97 |
| Jodessigsäure | 1 | 39 |
| 4-Chlormercurobenzoesäure | 0,1 | 69 |
| Natriumazid | 1 | 59 |
| Hydroxylamin | 1 | 37 |
| Phenylhydrazin | 1 | 8 |
| Semicarbazin | 1 | 82 |
| Tiron (Dinatriumsalz von 4,5 Di-hydroxy- | 1 | 110 |
| 1,3-benzoldisulfonsäure | | |
| o-Phenanthrolin | 1 | 89 |
| α-, α'-Dipyridyl | 1 | 100 |
| 8-Hydroxychinolin | 1 | 110 |
| EDTA (Ethylendiamintetraessigsäure) | 1 | 115 |
| Diethyldithiocarbamat | 1 | 89 |

### Beispiel 6

### Effekt von Methanol auf ruhende Zellen von NA40

Die Reaktion wurde 10 min. in der Gegenwart von 0 - 20 % (v/v) Methanol gemäss Tabelle 13 durchgeführt. Wie in Tabelle 14 gezeigt, wird die Aktivität durch die Zugabe von 5 - 15 % Methanol erhöht.

**Tabelle 13**

| **Reaktion mit ruhenden Zellen** | | | | | |
|---|---|---|---|---|---|
| | Methoden | | | | |
| | ① | ② | ③ | ④ | ⑤ |
| 1,0 M 3-Cyanpyridin | 1,0 ml | 1,0 ml | 1,0 ml | 1,0 ml | 1,0 ml |
| 0,1 M KPB* (pH 7,0) | 0,9 ml | 0,8 ml | 0,7 ml | 0,6 ml | 0,5 ml |
| Methanol | - | 0,1 ml | 0,2 ml | 0,3 ml | 0,4 ml |
| Zellsuspension (0,388 mg/ml) | 0,1 ml | 0,1 ml | 0,1 ml | 0,1 ml | 0,1 ml |
| Gesamtvolumen | 2,0 ml | 2,0 ml | 2,0 ml | 2,0 ml | 2,0 ml |

| | | | | | |
|---|---|---|---|---|---|
| * KPB = Kalimphosphatpuffer | | | | | |

**Tabelle 14**

| **Methanol-Effekt auf Amycolatopsis NA40** | | |
|---|---|---|
| **Methoden** | **Methanol [% (v/v)]** | **Relative Aktivität [%]** |
| ① | 0 | 100 |
| ② | 5 | 123 |
| ③ | 10 | 128 |
| ④ | 15 | 130 |
| ⑤ | 20 | 105 |

### Beispiel 7

### Anreicherung von Mikroorganismen der Gattung Rhodococcus

Verschiedene Bodenproben wurden im Anreicherungsmedium gemäss Tabelle 1 mit Cyanessigsäure als C- und N-Quelle angeimpft und entsprechend zu Beispiel 1 wurden die Mikroorganismen Rhodococcus GF270 und GF376 isoliert.

### Beispiel 8

### Biotransformation mit Mikroorganismen der Gattung Rhodococcus

### (1) Hitzestabilität der Mikroorganismen Rhodococcus GF270 und Rhodococcus GF376 im Vergleich zu Rhodococcus rhodochrous J1.

Zur Bestimmung der Hitzestabilität wurden die oben beschriebenen Mikroorganismen in folgenden Medien kultiviert. Rhodococcus rhodochrous J1 wurde 72 h lang in dem in der EP-A 0 307 926 beschriebenen Medium kultiviert. Die Mikroorganismen Rhodoccocus GF270 und GF376 wurden in folgenden Medien bei pH 7,0 bis zu 96 h kultiviert: Rhodococcus GF270 in einem Medium mit Hefeextrakt 12,5 g/l, Natriumcitrat 5,0 g/l, Methacrylamid 7,5 g/l, KH₂PO₄ 2,0 g/l MgSO₄ · 7 H₂O 0,5 g/l und CoCl₂·6 H₂O 30,0 mg. Rhodococcus GF376 in einem Medium mit Hefeextrakt 1,0 g/l, Natriumcitrat 10,0 g/l, Malzextrakt 15,0 g/l, Butyramid 7,5 g/l, KH₂PO₄ 2,0 g/l MgSO₄ · 7 H₂O 0,5 g/l und CoCl₂ · 6 H₂O 15,0 mg.
Dann wurden die ruhenden Zellen 15 min. bei verschiedenen Temperaturen bebrütet und danach wurde die bleibende Aktivität unter den Standardreaktionsbedingungen gemäss Beispiel 2(1) bestimmt.

Rhodococcus GF376 wies bis zu 50°C eine 100 %ige relative Aktivität, bei 60°C 70 %ige relative Aktivität und bei 70°C nahezu noch 5% relative Aktivität auf. Rhodococcus GF270 wies bis zu 60 °C eine relative Aktivität von nahezu 100 % und ebenso bei 70°C noch 5% relative Aktivität auf. Im Vergleich dazu hatte Rhodoccus rhodochrous J1 bis zu 50 °C eine 100 %ige relative Aktivität, bei 60 °C 80 % und bei 70°C keine Aktivität mehr.

Zusammengefasst kann daher festgehalten werden, dass Rhodoccus GF270 und GF 376 eine bessere Hitzestabilität als J1 aufwiesen und GF 270 die beste Hitzestabilität aufwies.

### (2) pH-Optimum der Rhodococcus-Stämme

Der Einfluss des pH-Wertes auf die Nitrilhydratase-Aktivität der Rhodococcus-Stämme GF270 und GF376 wurde, wie im Beispiel 2 (5) beschrieben, durchgeführt.
Das pH-Optimum von Rhodococcus GF270 lag bei pH 6-7,0 und für GF376 bei pH 6-8.

### (3) Substratspezifität der Rhodococcus-Stämme

Die Substratspezifität ist als relative Aktivität in Tabelle 15 zusammengefasst.

### (4) Akkumulation von Nikotinamid der Rhodococcus-Stämme

Analog zu Beispiel 2 (6) wurden die Rhodococcus-Stämme GF270 und GF376 mit 3-Cyanpyridin (ca. 500 mM) kultiviert. Dabei bildete Rhodococcus GF270 innerhalb 20 h ca. 8,5 M Nikotinamid und GF376 innerhalb 20 h 7,5 M Nikotinamid.

### (5) Toleranz von 3-Cyanpyridin auf die Aktivität der Rhodococcus-Stämme

Um die Toleranz von 3-Cyanpyridin zu testen, wurden ruhende Zellen 15 min lang bei 20 °C bei Konzentrationen von 3-Cyanpyridin zwischen 1 und 10 % (w/v) inkubiert und dann die Zellen abzentrifugiert. Nach Waschen der Zellen mit 0,85 % NaCl wurde die verbleibende Aktivität gemessen.

Die Toleranz von 3-Cyanpyridin als Substrat wurde bei verschiedenen Substratkonzentrationen getestet. Festgestellt wurde, dass bei einer Substratkonzentration von 2 % (w/v) die Nitrilhydratase-Aktivität von Rhododoccus rhodochrous J1 um den Faktor 1,4 abnahm, die Nitrilhydratase-Aktivität von Rhodococcus GF270 bei einer Substratkonzentration von 4 % (w/v) um den Faktor 1,17 abnahm und die Nitrilhydratase-Aktivität von Rhododoccus GF376 bei einer Substratkonzentration von 10 % (w/v) um den Faktor 1,25 abnahm.

Im Vergleich zu den anderen Rhodococcus-Stämmen wies Rhodococcus rhodochrous J1 die schlechteste Toleranz für 3-Cyanpyridin auf.

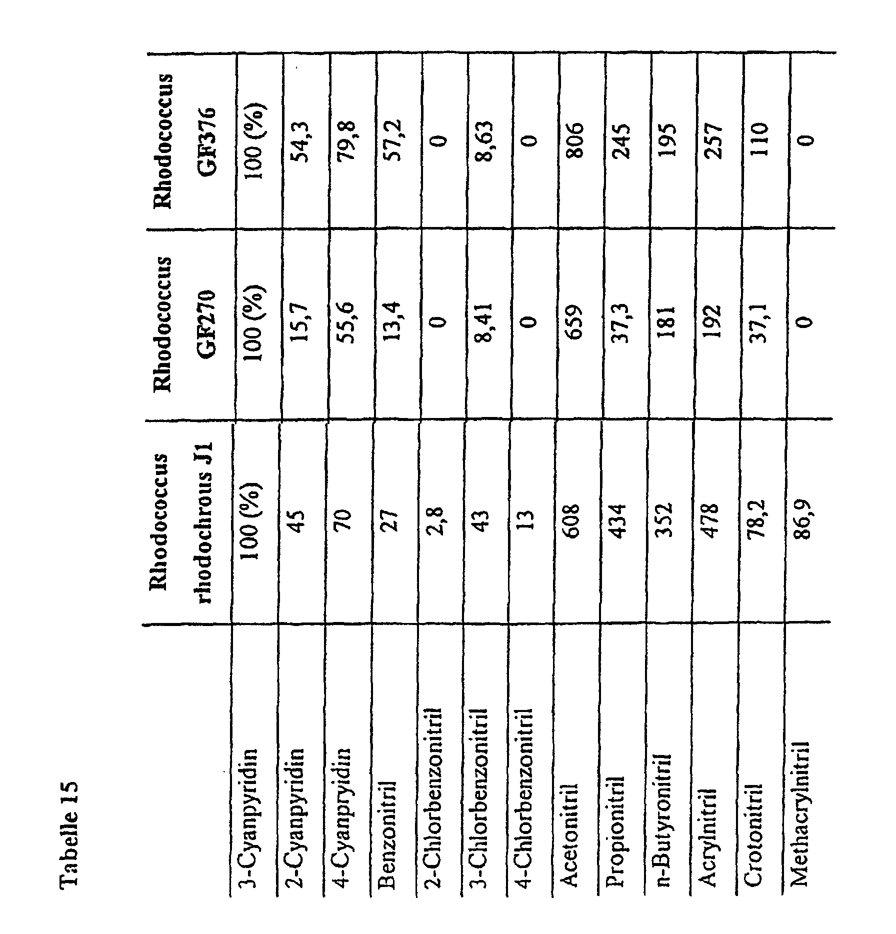

## Patentansprüche

1. Mikroorganismen der Gattung Amycolatopsis oder Actinomadura, **dadurch gekennzeichnet, dass** sie befähigt sind, ein Nitril in ein Amid umzusetzen, sowie Enzymextrakte daraus.

2. Mikroorganismen nach Anspruch 1 der Spezies Amycolatopsis NA40 und Amycolatopsis NE31 wie hinterlegt unter den Hinterlegungsnummern DSM 11617 und DSM 11616, sowie deren funktionell äquivalente Varianten und Mutanten.

3. Mikroorganismen der Spezies Rhodococcus GF270 und GF376 wie hinterlegt unter den Hinterlegungsnummern DSM 12211 und DSM 12175, sowie deren funktionell äquivalente Varianten und Mutanten, **dadurch gekennzeichnet, dass** sie befähigt sind, ein Nitril in ein Amid umzusetzen, sowie Enzymextrakte daraus.

4. Enzym mit Nitrilhydratase-Aktivität, erhältlich aus den Mikroorganismen gemäss den Ansprüchen 1 und 2.

5. Enzym nach Anspruch 4, **gekennzeichnet durch**
a) ein pH-Optimum von pH 6,5 ± 1,0
b) ein Temperatur-Optimum zwischen 35 und 40 °C bei einem pH von 7,0
c) einen K_{M}-Wert für das Substrat von 3-Cyanpyridin von 41,7 mM ± 7,7 mM.

6. Verfahren zu Herstellung von Amiden, **dadurch gekennzeichnet, dass** man ein Nitril, als Substrat, mittels den Mikroorganismen gemäss einem der Ansprüche 1 bis 3, mit einem Enzymextrakt von diesen Mikroorganismen oder mittels dem Enzym nach Anspruch 4 zum entsprechenden Amid umsetzt.

7. , Verfahren nach Anspruch 6, **dadurch gekennzeichnet, dass** als Nitrit ein gegebenenfalls substituiertes aliphatisches Nitril mit 1 bis 10 Kohlenstoffatomen eingesetzt wird.

8. Verfahren nach Anspruch 6, **dadurch gekennzeichnet, dass** als Nitril ein gegebenenfalls substituiertes aromatisches Nitril mit 4 bis 10 Kohlenstoffatomen im aromatischen Ringsystem eingesetzt wird.

9. Verfahren nach Anspruch 8, **dadurch gekennzeichnet, dass** das aromatische Nitril ausgewählt ist aus den Verbindungen der allgemeinen Formeln 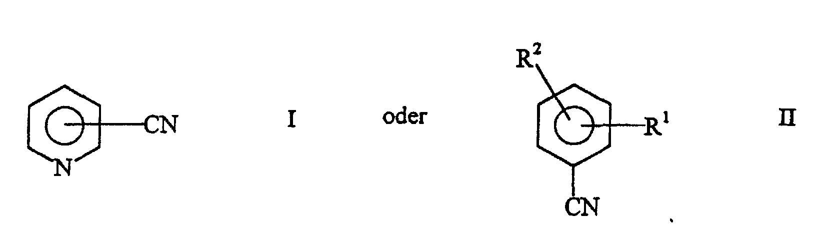 worin R¹ und R² ein Wasserstoffatom, ein Halogenatom oder C₁₋₄-Alkyl bedeuten.

10. Verfahren nach einem der Ansprüche 6 bis 9, **dadurch gekennzeichnet, dass** man die Umsetzung bei einer Temperatur von 0 bis 50 °C und bei einem pH von 4,5 bis 10 durchführt.

11. Verfahren nach einem der Ansprüche 6 bis 10, **dadurch gekennzeichnet dass** man die Umsetzung mittels Mikroorganismen der Gattung Amycolatopsis mit der Bezeichnung NA40 (DSMZ 11617) oder NE31 (DSMZ 11616) oder mit deren funktionell äquivalenten Varianten und Mutanten durchführt.

## Claims

1. Microorganisms of the Amycolatopsis or Actinomadura genus, **characterised in that** they are capable of converting a nitrile into an amide, and enzyme extracts thereof.

2. Microorganisms as claimed in claim 1, of the Amycolatopsis NA40 and Amycolatopsis NE31 species as registered under registration numbers DSM 11617 and DSM 11616, and their variants and mutants having equivalent functions.

3. Microorganisms of the Rhodococcus GF270 and GF376 species as registered under registration numbers DSM 12211 and DSM 12175, as well as their variants and mutants having equivalent functions, **characterised in that** they are capable of converting a nitrile into an amide, and enzyme extracts thereof.

4. Enzyme with nitrile hydratase activity, which can be obtained from microorganisms as claimed in claims 1 and 2.

5. Enzyme as claimed in claim 4, **characterised by**
a) a pH optimum of pH 6.5 ± 1.0
b) a temperature optimum of between 35 and 40°C at a pH of 7.0
c) a K_{M} value for the 3-cyano-pyridine substrate of 41.7 mM ± 7.7 mM.

6. Method of producing amides, **characterised in that** a nitrile, constituting a substrate, is converted by means of microorganisms as claimed in one of claims 1 to 3, with an enzyme extract from these microorganisms, or by means of the enzyme as claimed in claim 4, into the corresponding amide.

7. Method as claimed in claim 6, **characterised in that** an optionally substituted aliphatic nitrile with 1 to 10 carbon atoms is used as the nitrile.

8. Method as claimed in claim 6, **characterised in that** an optionally substituted aromatic nitrile with 4 to 10 carbon atoms in the aromatic ring system is used as the nitrile.

9. Method as claimed in claim 8, **characterised in that** the aromatic nitrile is selected from compounds having the general formulae 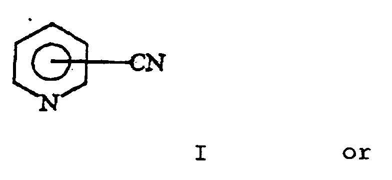 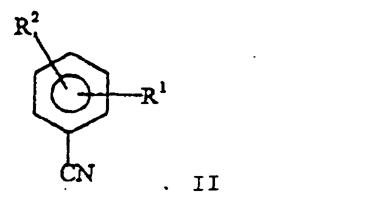 in which R¹ and R² stand for a hydrogen atom, a halogen atom or C₁₋₄ alkyl.

10. Method as claimed in one of claims 6 to 9, **characterised in that** the conversion is operated at a temperature of from 0 to 50°C and at a pH of from 4.5 to 10.

11. Method as claimed in one of claims 6 to 10, **characterised in that** the conversion is effected by means of microorganisms of the Amycolatopsis genus denoted by NA40 (DSMZ 11617) or NE31 (DSMZ 11616) or with variants and mutants thereof having equivalent functions.

## Revendications

1. Microorganismes du genre Amycolatopsis ou Actinomadura, **caractérisés en ce qu'**ils sont aptes à transformer un nitrile en un amide, ainsi que les extraits enzymatiques obtenus à partir de ces microorganismes.

2. Microorganismes selon la revendication 1 des espèces Amycolatopsis NA40 et Amycolatopsis NE31, tels que déposés sous les numéros de dépôt DSM 11617 et DSM 11616, ainsi que leurs variants et mutants fonctionnellement équivalents.

3. Microorganismes des espèces Rhodococcus GF270 et GF376, tels que déposés sous les numéros de dépôt DSM 12211 et DSM 12175 ainsi que leurs variants et mutants fonctionnellement équivalents, **caractérisés en ce qu'**ils sont aptes à transformer un nitrile en un amide, ainsi que les extraits enzymatiques obtenus à partir de ces microorganismes.

4. Enzyme présentant une activité nitrile-hydratase, susceptibles d'être obtenue à partir des microorganismes selon les revendications 1 et 2.

5. Enzyme selon la revendication 4, **caractérisée par**
a) un optimum de pH de 6,5 ± 1,0
b) un optimum de température entre 35 et 40°C à un pH de 7,0
c) une valeur Kₘ pour le substrat de 3-cyanopyridine de 41,7 mM ± 7,7 mM.

6. Procédé pour la préparation d'amides, **caractérisé en ce qu'**on transforme un nitrile, en tant que substrat, au moyen des microorganismes selon l'une quelconque des revendications 1 à 3, avec un extrait enzymatique de ces microorganismes ou au moyen de l'enzyme selon la revendication 4 en amide correspondant.

7. Procédé selon la revendication 6, **caractérisé en ce qu'**on utilise comme nitrile un nitrile aliphatique le cas échéant substitué comprenant de 1 à 10 atomes de carbone.

8. Procédé selon la revendication 6, **caractérisé en ce qu'**on utilise comme nitrile un nitrile aromatique le cas échéant substitué comprenant de 4 à 10 atomes de carbone dans le système cyclique aromatique, ou noyau aromatique.

9. Procédé selon la revendication 8, **caractérisé en ce que** le nitrile aromatique est choisi parmi les composés de formules générales 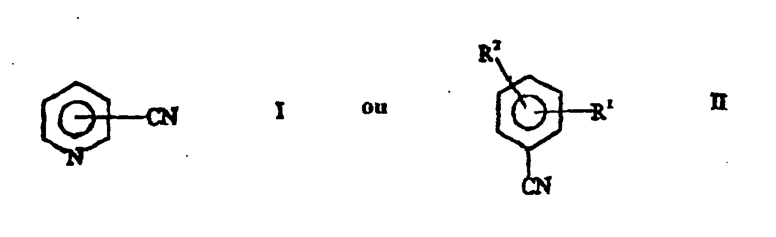 dans lesquelles R¹ et R² représentent un atome d'hydrogène, un atome d'halogène ou un alkyle en C₁₋₄.

10. Procédé selon l'une des revendications 6 à 9, **caractérisé en ce qu'**on effectue la transformation à une température de 0 à 50°C et à un pH de 4,5 à 10.

11. Procédé selon l'une des revendications 6 à 10, **caractérisé en ce qu'**on effectue la transformation au moyen de microorganismes du genre Amycolatopsis désignés par NA40 (DSMZ 11617) ou NE31 (DSMZ 11616) ou avec les variants et mutants fonctionnellement équivalents de ces microorganismes.
